# EUROPEAN PATENT SPECIFICATION

(11) **EP 4 429 584 B1**
(45) Date of publication and mention of the grant of the patent: **11.11.2026**
(21) Application number: 22890523.8
(22) Date of filing: 07.11.2022
(51) Int. Cl.: A61B 50/36, A61M 5/32, B29C 45/00, B65D 43/02, B65F 1/16, B29C 45/72, B29C 35/16

(54) **REINFORCED CLINICAL WASTE CONTAINER COMPRISING WOOD FIBRES AND METHOD FOR PRODUCING THE SAME**
VERSTÄRKTER BEHÄLTER FÜR KLINISCHE ABFÄLLE MIT HOLZFASERN UND HERSTELLUNGSVERFAHREN DAFÜR
RÉCIPIENT DE DÉCHETS CLINIQUES RENFORCÉ COMPRENANT DES FIBRES DE BOIS ET SON PROCÉDÉ DE PRODUCTION

(30) Priority: 08.11.2021 SE 2151368
(43) Date of publication of application: 18.09.2024
(73) Proprietor: FrostGreen AB, 182 53 Danderyd (SE)
(72) Inventor: WALL, Peter, 633 50 Eskilstuna (SE)
(74) Representative: Noréns Patentbyrå AB
(86) International application number: PCT/SE2022/051029
(87) International publication number: WO 2023/080833

(56) References cited:
- EP-A1- 1 380 316
- CN-A- 111 110 362
- JP-A- H07 184 961
- US-A- 3 282 477
- US-A- 4 288 066
- US-A- 4 502 606
- US-A- 4 520 926
- US-A- 4 520 926
- US-A- 5 249 680
- US-A- 5 249 680
- US-A- 5 941 385
- US-A- 5 941 385
- US-A- 6 062 001
- ANONYMOUS: "Kanylburk 2,2 liter", APOTEA, 9 August 2020 (2020-08-09), pages 1 - 3, XP093065560, Retrieved from the Internet <URL:http://web.archive.org/web/20200809205554/https://www.apotea.se/kanylburk-2.2-liter> [retrieved on 20230720]

## Description

The present invention relates to a clinical waste container and to a method for producing the same. Such container can be for disposing needles and other sharp waste (a so called sharps container), or for other clinical/medical waste.

Clinical or medical waste, such as used syringe needles, constitute a hazard and needs to be handled according to certain safe protocols. Normally, hospital personnel and other care-givers put such waste material in dedicated containers, in the form of lidded buckets or baskets, for transport to a facility and subsequent destruction.

Various examples of such waste container are shown in documents US 4 502 606 A, US 5 941 385 A, US 4 520 926 A, US 5 249 680 A, XP093065560, CN 111 110 362 A, US 6 062 001 A and EP 1 380 316 A1.

Normally, the container is a disposable container, intended for one-time use only.

A number of requirements are put on such containers:
They need to be sufficiently resistant to externally applied abrasive wear and violent force, and also so that disposed waste in the container does not run the risk of protruding out from the container.

They need to be stackable in several stories without breaking.

They need to be available in standard volume sizes.

They need to comprise a lid that can be removed but that will not come off by accident during handling.

They need to provide an interface (such as a needle or blade disconnector) allowing care-givers to quickly and safely dispose of syringe needles and other medical waste material using only one hand. This may include disposing of such a needle by holding the syringe and twisting or turning it, using the lid as a resistance, so that the needle comes loose, in turn subjecting the lid to bending forces that it must be able to withstand.

They need to be sealable both temporarily and permanently.

They need to be completely self-contained, meaning that no loose parts should be present that can be lost during handling.

They need to be manufacturable in a simple process having high throughput.

Since such containers are manufactured in large volumes, it is important that the cost of producing them is low.

Conventionally, such containers are manufactured from plastic material, such as polypropylene. This is an inexpensive material that can be injection moulded to a desired shape, forming a durable container.

However, in order to lower the climate footprint associated with such containers, it is desirable to produce them using a material that carries lower amounts of fossil-sourced carbon. Destruction is typically via incineration, and re-use or recycling is typically not possible.

It has turned out to be difficult to achieve such a container in such material, while still fulfilling all the above requirements.

The present invention solves the above described problems, and proposes to manufacture a clinical waste container using a bio-composite material, especially a material being a mixture of wooden fibres and a plastic material. More particularly, the present invention proposes to arrange such a clinical waste container in specific ways so as to fulfil said requirements.

Hence, the invention relates to a clinical waste container being associated, in an upright operating orientation of the clinical waste container, with an upwards axial direction, an outwards radial direction and an angular direction, the clinical waste container comprising a lower basket part, having an upper basket opening; an upper lid part, arranged to be fastened to said basket part so as to cover said basket opening, the lid part comprising a cylindrical part having an upper lid opening arranged to receive clinical waste through said lid opening into said basket part; and a lid cap part, arranged to be fastened to said lid part so as to cover said lid opening, the lid cap part comprising a cylindrical part having a closed upper end and an inner dimension being larger than an outer dimension of said cylindrical part of the lid part, the cylindrical part of the lid cap part being arranged to be slid onto the cylindrical part of the lid part so that the lid cap part as a result covers the lid opening, wherein the cylindrical part of the lid cap part comprises inner snap-lock means arranged to engage with outer snap-lock means arranged on the cylindrical part of the lid part so as to fasten the lid cap part on the lid part by the material of at least the lid cap part deforming elastically into a snap-fit lock, which container is characterised in that the inner snap-lock means comprises several separated elongated protrusions, running in the angular direction in relation to the lid cap part and together defining a broken circle, in that the outer snap-lock means comprises an elongated recess, running in the angular direction in relation to the lid part and being arranged to, by at least the material of the lid cap part elastically deforming, engage with said protrusions by said protrusions protruding into said recess along a respective angular extension of said protrusions and said recess, respectively, so as to form a first snap-fit lock, in turn fastening the lid cap part to the lid part, in that the lid part comprises at a lower end of its cylindrical part a bend at which the cylindrical part of the lid part changes direction at least 60° as seen in a cross-section including a centre axially extending axis of the cylindrical part of the lid part, the bend having an inner radius of curvature of at the most 5 mm and an outer radius of curvature of at the most 5 mm, in that the material thickness of the lid part is at least 25% thicker locally at said bend as compared to a material thickness adjacent to the bend in said cylindrical part of the lid part, and in that the lid part and the lid cap part are made from a plastic material comprising at least 30% by volume wooden fibres.

Moreover, the invention relates to a method for producing a clinical waste container of said type, wherein the method comprises providing a metal mould and a heated nozzle; injecting molten plastic material comprising at least 30% by volume wooden fibres via said heated nozzle into said mould; allowing the plastic material to harden by cooling to form said lid part; removing the lid part from the mould; and providing said basket part.

In the following, the invention will be described in detail, with reference to exemplifying embodiments of the invention and to the enclosed drawings, wherein:

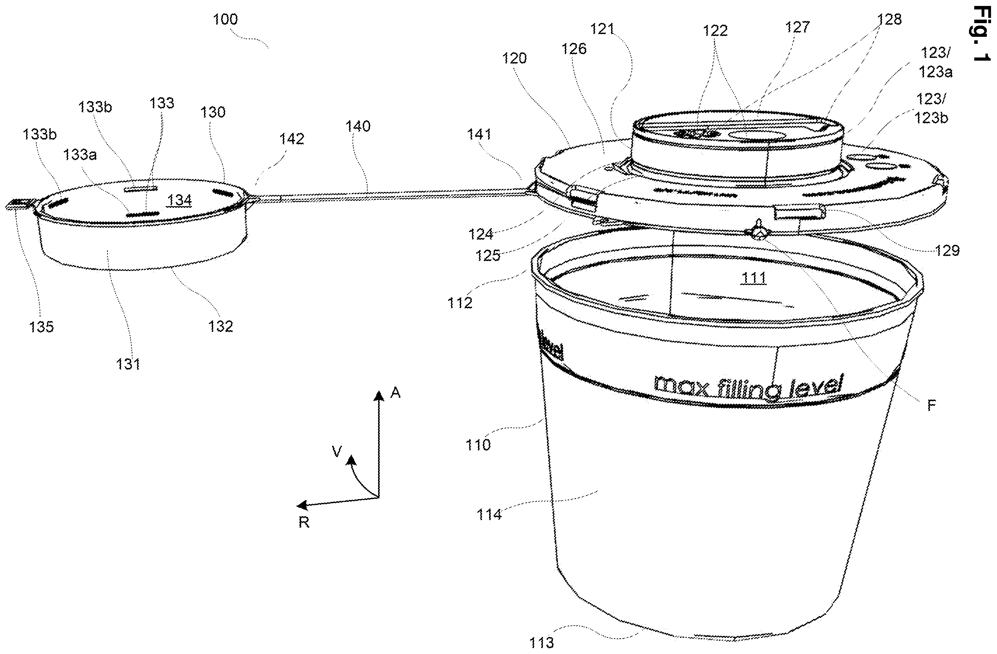

Figure 1 is a perspective view of a first clinical waste container 100 according to the present invention, including a basket part 110 and a lid part 120. The view in Figure 1 is partly exploded. It is noted that the lid part 120 is connected to a lid cap part 130, via a connecting part 140 that is shown as straight in Figure 1. The connecting part 140 is moulded in this straight orientation, but during use the connecting part 140 may be bent 180° so that the lid cap part 130 is brought, upside down, onto the lid part 120 so as to seal a lid opening 122 of the lid part 120. Herein, an operating orientation is referred to, in which the clinical waste container 100 is in an upright position with the lid part 120 mounted on the basket part 110. This orientation is shown (albeit the lid part 120 exploded upwards in relation to the basket part 110) in Figure 1. It is then noted that the connecting part 140 may or may not, in this operating orientation, be bent so as to allow the lid cap part 130 to reach and cover the lid opening 122. A coordinate system A, R, V is shown in Figure 1 for reference.
Figures 2 and 3 are similar to Figure 1, but each show the clinical waste container 100 from a different respective perspective.
Figure 4 is also similar to Figure 1, but shows the clinical waste container 100 partly removed, so that a substantially radially extending cross-section being exposed.
Figure 5 is a detail view of the clinical waste container 100 shown in Figure 4, having the same cross-section exposed.
Figure 6 is a simplified cross-sectional view of a bend 125 of the clinical waste container 100.
Figure 7 shows a detail of the lid cap part 130 of the clinical waste container 100, partly removed along a different cross-section.
Figure 8 is a detail of the clinical waste container 100, showing the connecting part 140.
Figure 9 is a perspective view similar to Figure 1, but partly removed. Everything below a contact plane 223 has been removed. The contact plane 223 is a plane of contact between two mould parts 221, 222 (not shown in Figure 9) that together define a mould 220 for making the lid part 120 and the lid cap part 130 as a single, integrated material body, interconnected via the connecting part 140, using injection moulding in a method accordin to the present invention.
Figure 10 is a side view of the lid part 120 and the lid cap part 130, further showing said contact plane 223.
Figure 11 illustrates, in a perspective view, an alternative assembly 300 including a lid part 320, a lid cap part 330 and a connecting part 340.
Figure 12 is a cross-sectional view illustrating the connecting part 140.
Figure 13 is a partly removed, cross-sectional view of a mould 200. Figure 13 shows a mould 200 for making the basket part 110 in a method according to the present invention, but it is realized that a similar mould, perhaps having more than one injection molten plastic point 225, may be used to produce the integrated assembly comprising the lid part 120, the lid cap part 130 and the connecting part 140.
Figure 14 is a flow chart illustrating a method according to the present invention.

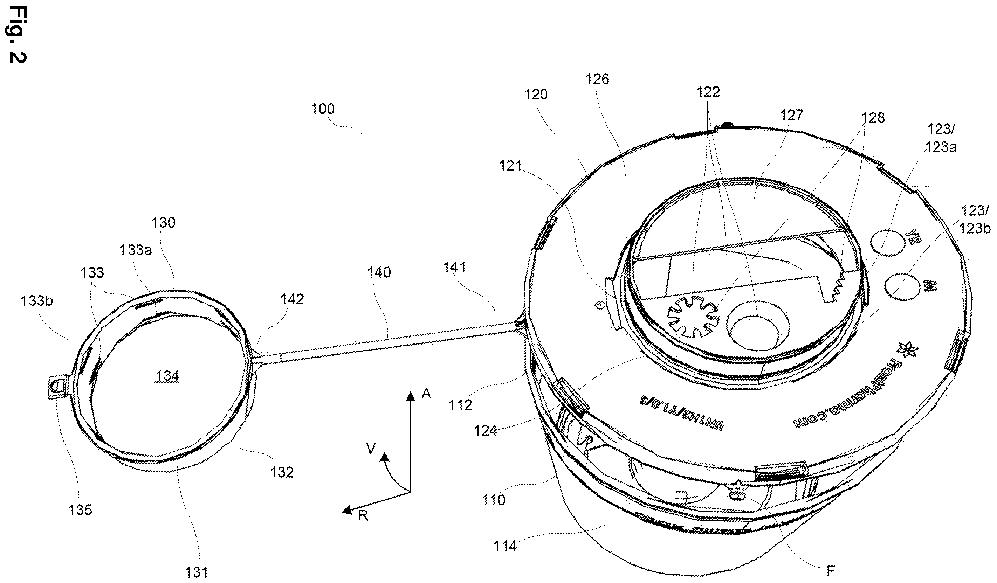

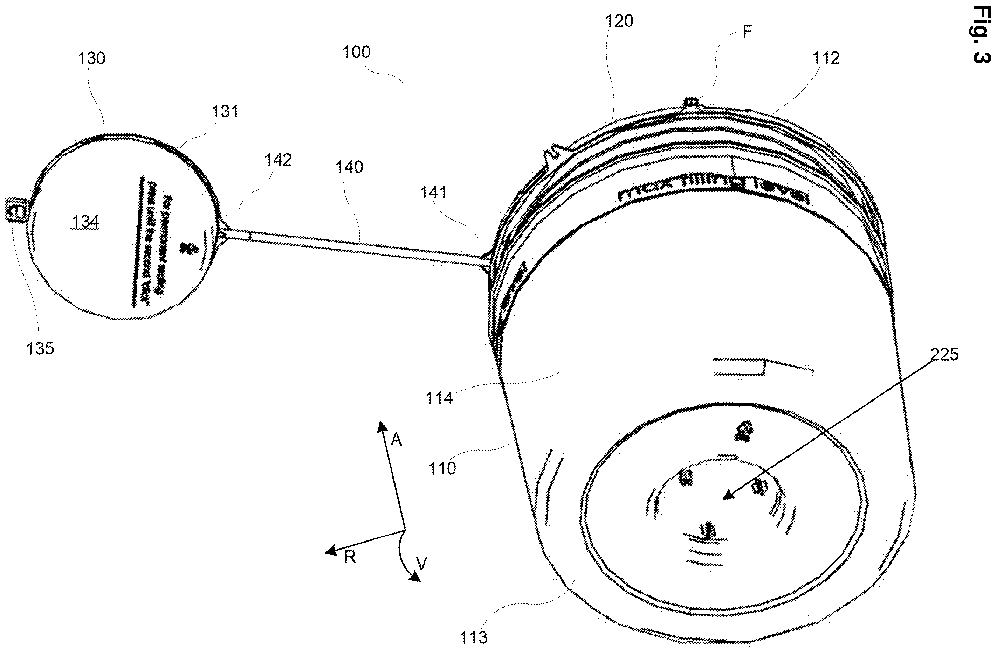

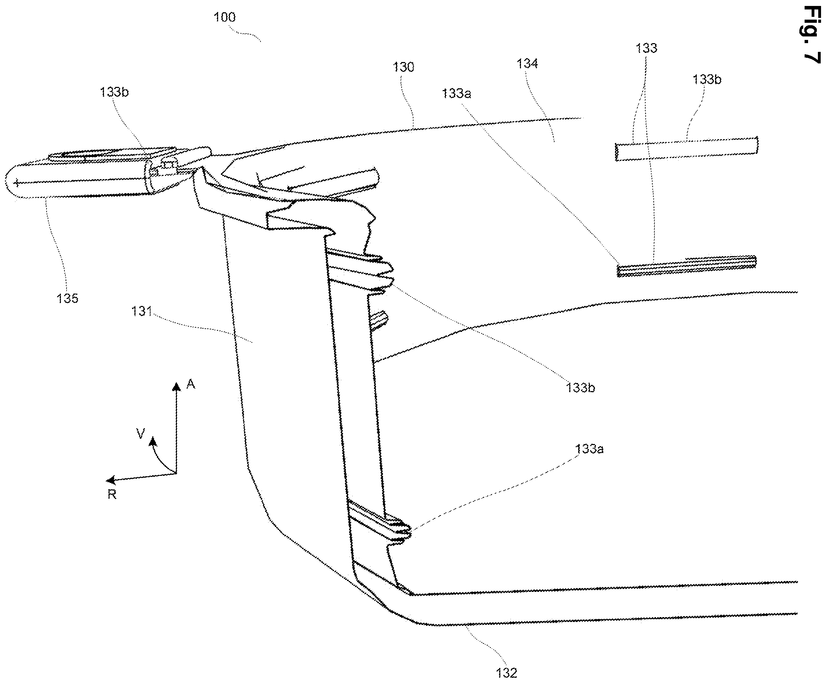

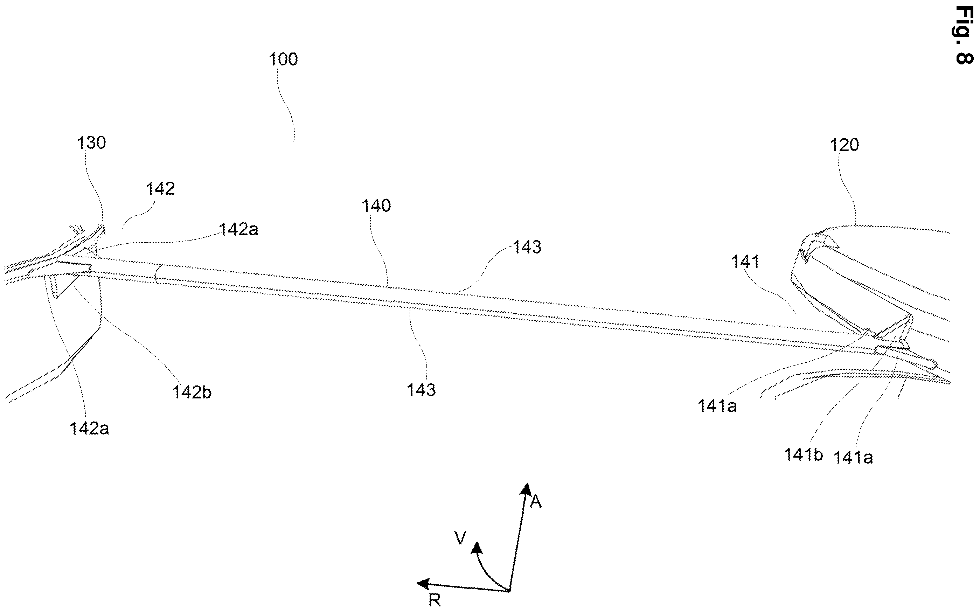

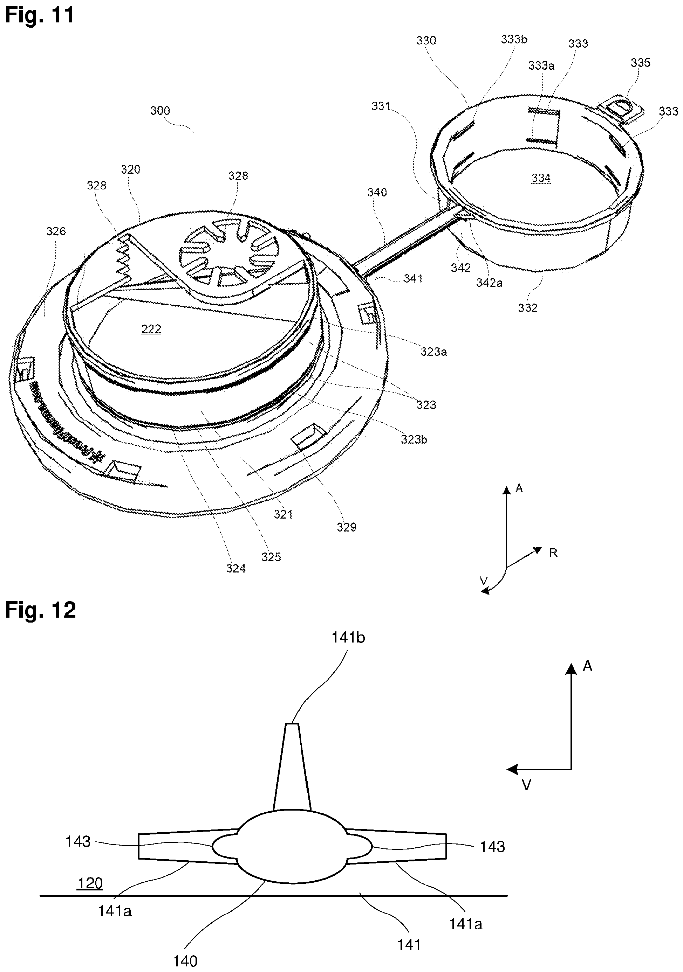

All Figure share the same reference numerals for same or corresponding parts. Figure 11 uses the same last digits in the reference numerals as Figures 1-10 and 12.

Hence, with general reference to the Figures, a clinical waste container 100 is associated, in said upright operating orientation of the clinical waste container 100, with an upwards axial direction A, an outwards radial direction R and an angular direction V. When referring to "upwards" and "downwards" directions for the lid cap part 130, it is assumed that the lid cap part is put onto the lid part 120 (i.e. the connecting part 140 is bent 180°).

The clinical waste container 100 comprises the lower basket part 110, in turn having an upper basket opening 111. The basket part 110 also has a tapered or straight side wall 114.

The clinical waste container 100 also comprises the upper lid part 120, in turn being arranged to be fastened to said basket part 110 so as to cover said basket opening 111. The lid part 120 further comprises a cylindrical part 121 having an upper lid opening 122 arranged to receive clinical waste through said lid opening 122 into said basket part 110.

Even though the clinical waste container 100 is shown as generally circular symmetric in a cross-section taken perpendicularly to the axial direction A, it is realised that the clinical waste container 100 may have other general shapes, such as cross-sectionally square or rectangular, as the case may be. The corresponding also applies to the complementary-shaped cylindrical parts 121, 131 discussed herein. Hence, the cylindrical parts 121, 131 may each be circular-symmetric cylinders or have any other suitable cylindrical shape, as long as they are complementarily shaped so as to achieve a snug fit when the lid cap part 130 is mounted onto the lid part 120 so as to seal the container 100 temporarily or permanently (see below).

Everything which is said regarding the clinical waste container 100 is equally applicable to the assembly 300, as well as to the method of the present invention, and vice versa, as applicable.

The clinical waste container 100 further comprises said lid cap part 130, in turn being arranged to be fastened to said lid part 120 so as to cover said lid opening 122. The lid cap part 130 comprises said cylindrical part 131 having a closed upper end 132 and an inner dimension being larger than an outer dimension of said cylindrical part 121 of the lid part 120. The cylindrical part 131 of the lid cap part 130 is arranged to be slid onto the cylindrical part 121 of the lid part 120 so that the lid cap part 130 as a result covers the lid opening 122. In other words, the cylindrical part 131 of the lid cap part 130 is arranged to at least partly cover the cylindrical part 121 of the lid part 120 when the lid cap part 130 is fully (permanently, see below) mounted onto the lid part 120.

The cylindrical parts 121, 131 may both be slightly cone-shaped to facilitate the sliding of the cylindrical part 131 onto the cylindrical part 121.

The cylindrical part 131 of the lid cap part 130 comprises inner snap-lock means 133, in turn being arranged to engage with outer snap-lock means 123 arranged on the cylindrical part 121 of the lid part 120 so as to fasten the lid cap part 130 on the lid part 120 by the material of at least the lid cap part 130 deforming elastically into a snap-fit lock.

According to the present invention, the lid part 120 and the lid cap part 130 are made from a plastic material comprising at least 30% by volume wooden fibres. The lid part 120 may be made from the same material as the lid cap part 130, in particular in case they are injection moulded as one single, integrated part. The basket 110 may also be made from a plastic material comprising wooden fibres, such as at least 40% by volume wooden fibres.

The remaining material, apart from the wooden fibres, may be a conventional plastic material, such as a conventional thermoplastic, such as polypropylene, or a mixture of such a plastic material with one or several other plastic materials and/or additives. More generally, the remaining material may be any suitable polymer, such as a virgin fossil- based, a recycled fossil-based or a biobased polymer. It is realised that many different such polymers may be suitable for a container of the present type, acting as reinforcement and providing sufficiently elastic, strong and abrasive-resistant properties to the composite formed when admixing wooden fibres.

Hence, the material used to manufacture the present clinical waste container 100 is a bio-composite material. As an example, a material of the type DuraSense^{®}, marketed by StoraEnso, Sweden, may be used.

Using such a material, the carbon dioxide load of the clinical waste container 100 is significantly reduced, which is advantageous from an environmental point of view.

However, such a material is also significantly less elastic and more brittle as compared to, for instance, polypropylene being a material conventionally used for medical waste containers.

This makes it difficult to design such a clinical waste container 100 that can fulfil all the initially stated requirements on clinical waste containers. This is important, not least since they are often used to transport medical risk waste such as sharp needles and contaminated material.

The present inventors have developed a series of specific design principles that allow a clinical waste container 100 to be manufactured using a biomaterial of the type discussed above without resulting in a quality-wise inferior product.

In the following, these design principles will be described in detail.

Regarding first said inner snap-lock means 133, they may comprise several separated elongated protrusions 133a, 133b, running in the angular direction V in relation to the lid cap part 130 and together defining a broken circle. In other words, there are several discrete such protrusions (in the Figures both an upper series of such discrete protrusions 133a and a lower series of such protrusions 133b), the protrusions belonging to one and the same broken circle being interrupted by stretches without any such protrusion.

Furthermore, the outer snap-lock means 123 may comprise an elongated recess 123a, 123b, running in the angular direction V in relation to the lid part 120 and being arranged to, by at least the material of the lid cap part 130 elastically deforming, engage with said protrusions 133a, 133b by said protrusions 133a, 133b protruding into said recess 123a, 123b along a respective angular V extension of said protrusions 133a, 133b and said recess 123a, 123b, respectively, so as to form a first snap-fit lock, in turn fastening the lid cap part 130 to the lid part 120. In contrast to protrusions 133a, 133b of the inner snap-lock means 133, the elongated recesses 123a, 123b may extend uninterrupted along the full 360° lap in the angular direction V. However, as discussed each of the protrusions 133a, 133b will be interrupted along the angular direction V at least on one location, preferably in at least two locations or even at least in four locations. In the Figures, a preferred embodiment in which each of the protrusions 133a, 133b is interrupted in six locations is shown.

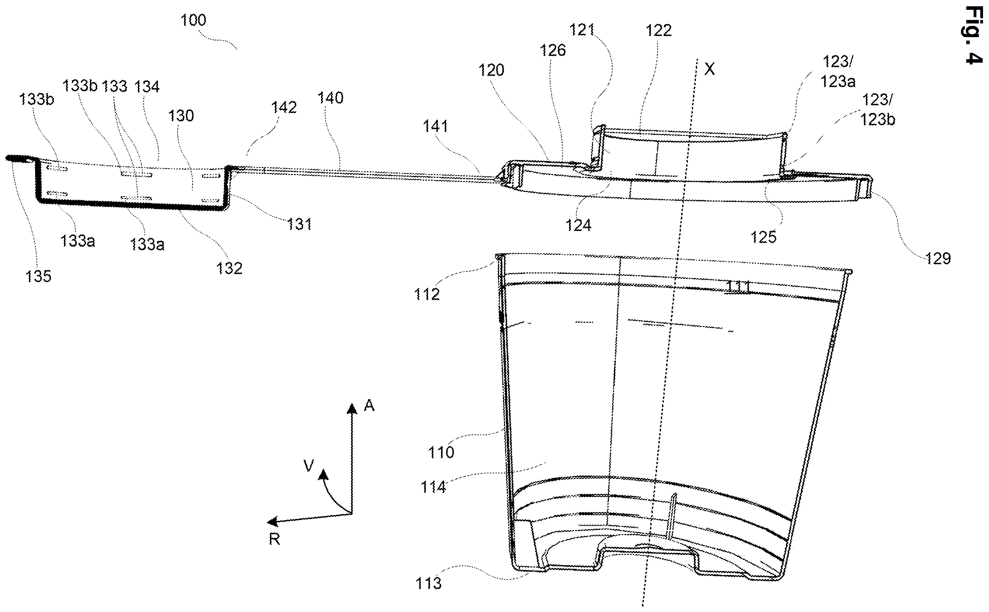

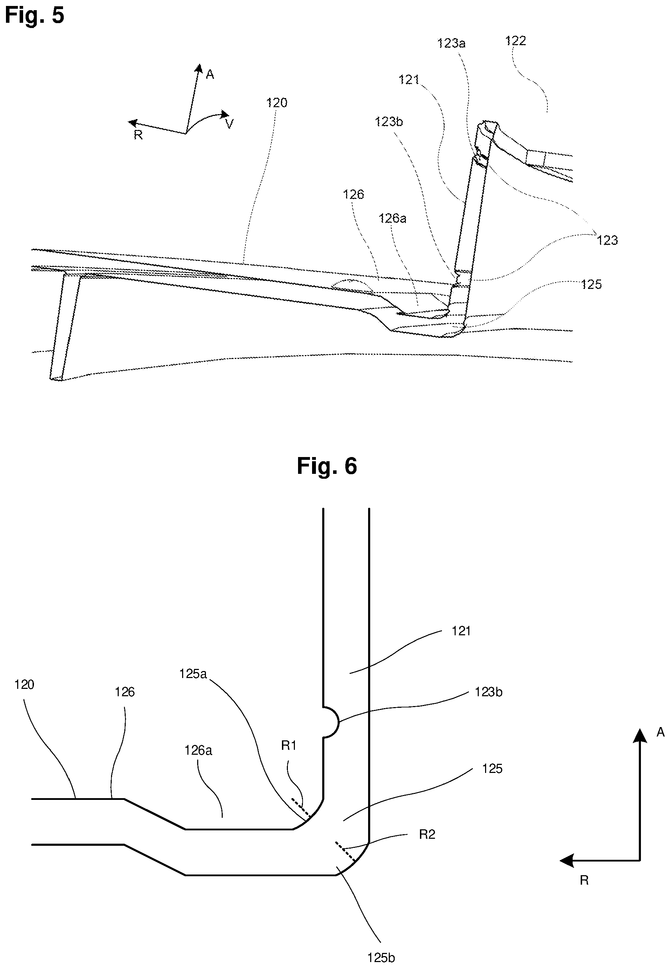

The lid part 120 may comprise, at a lower end 124 of its cylindrical part 121, a bend 125 (see Figures 5 and 6) at which bend 125 the cylindrical part 121 of the lid part 120 changes direction at least 60°, such as at least 80°, such as at least locally 85-95°, as seen in a cross-section including an axially A extending centre axis X (see Figure 4) of the cylindrical part 121 of the lid part 120. An example of such a cross-section is in fact the cross-section at which Figure 4 is partly removed.

The bend 125 may have a constant cross-section along the full 360° radial direction, or at least along more than 50% of a full lap along the radial direction.

Moreover, the bend 125 may have an inner radius of curvature R1 (see Figure 6) of at the most 5 mm and an outer radius of curvature R2 of at the most 5 mm.

Furthermore, a material thickness, perpendicularly to opposite sides of the materially as measured locally, of the lid part 120 may be at least 25% thicker locally at said bend 125 as compared to a corresponding material thickness adjacent to the bend 125 in said cylindrical part 121 of the lid part 120.

The locally thickening of the material may be limited to the bend 125 itself, by simply adjusting the radii of curvature R1, R2 as described below, keeping the material thickness uniform or at least substantially uniform (taking into consideration local features, as the case may be) in the rest of the cylindrical part 121 and the cover 126 of the lid part 120.

The present inventors have realised that a design of the lid part 120 and the lid cap part 130 having such cooperating snap-lock means 123, 133, where the inner snap-lock means 133 are arranged in the way described above, forming a respective broken circle in the radial direction, allows for an effective snap-lock both for closing the container 100 temporarily and permanently, and that allows the lid cap part 130 to be injection moulded without being damaged when ejected from the mould once hardened. The local thickness of the bend 125 surprisingly is enough so as to strengthen the lid part 120 to be able to press down the lid cap part 130 with sufficient force so as to engage said snap-lock means 123, 133 with each other. By providing such an only locally increased thickness, the total amount of material used to manufacture the lid part 120 can be kept to a minimum, with a smaller material thickness in the rest of the cylindrical part 121 and the cover 126 than what would otherwise have been the case.

In other words, the presently described combination of the snap-lock means 123, 133 and the locally thicker bend 125 achieves a closing mechanism that works adequately in a lid part - lid part cap assembly that can be produced by rapid injection moulding and without having to use more material than what is necessary.

As mentioned, the material thickness of the lid part 120 at said bend 125 may be locally varied by the inner radius of curvature R1 being sufficiently large in relation to the outer radius of curvature R2. In a particularly simple design, this adaptation of radii of curvature is the only reason for the material thickness to be locally increased, and the respective material surfaces may then be flat outside of an inner curved bend part 125a and an outer curved bend part 125b.

An outside of the lid part 120 at said bend 125 (the outer bend part 125b) may have a part-circular shape in the above-discussed cross-section. Similarly, an inside of the lid part 120 at said bend 125 (the inner bend part 125a) may have a part-circular shape in said cross-section.

Concretely, the material thickness of the lid part 120 adjacent to said bend 125 may be at the most 2 mm, such as at the most 1.8 mm, such as between 1.5 and 1.7 mm, such as about 1.6 mm. The whole lid part 120 may have a substantially uniform material thickness (such as being the same across at least 50% of the total surface of the lid part 120), being as discussed in relation to the material thickness adjacent to the bend 125.

Said inner radius of curvature R1 may be at least 1.2 mm, such as at least 1.3 mm. The inner radius of curvature R1 may be at the most 2.0 mm, such as at the most 1.7 mm. In preferred examples, the inner radius of curvature R1 is about 1.5 mm.

Said outer radius of curvature R2 may be at least 1.7 mm, such as at least 1.8 mm. The outer radius of curvature R2 may be at the most 2.5 mm, such as at the most 2.2 mm. In preferred examples, the outer radius of curvature R2 is about 2.0 mm.

In particular, the material thickness of the lid part 120 at least adjacent to the bend 125, in the cylindrical part 121 and/or the cover 126, may be constant.

The cover 126 is shown in the Figures to be a generally flat part, extending substantially horizontally between the edge of the cylindrical part 121 to a peripheral edge of the lid part 120. It is understood that this cover 126 may have other shapes, but is arranged to cover the basket opening 111 outside of the cylindrical part 121.

In the Figures, the snap-lock means 123, 133 comprise two axially offset outer snap-lock means 123 as well as two axially offset inner snap-lock means 133. In various examples, there may be only one of either one or both of said snap-lock means 123, 133; and/or more than two of either one or both of said snap-lock means 123, 133. At any rate, it is preferred that an axially A lower-most one of the inner snap-lock means 133 is of the type mentioned above, forming a broken circle. It is also preferred that no inner snap-lock means 133 being arranged axially A above another inner snap-lock means 133 is provided as a full circle (360°) protrusion. In case there is only one inner snap-lock means 133, it is preferred that this inner snap-lock means 133 is arranged near the axially A lower end 124 when the lid cap part 130 is fully pushed down onto the lid part 120 cylinder 121, in other words such single inner snap-lock means 133 is provided near the lower opening 134 of the lid cap part 130.

However, and as is illustrated in the Figures, in preferred embodiments the outer snap-lock means 123 may comprise the pair of axially A offset but parallel elongated recesses 123a, 123b on the cylindrical part 121 of the lid part 120.

The inner snap-lock means 133 may comprise the pair of axially A offset but parallel elongated protrusions 133a, 133b on the cylindrical part 131 of the lid cap part 130.

As mentioned, the cylindrical part 131 of the lid cap part 130 may be arranged to be pressed down onto (around) the cylindrical part 121 of the lid part 120 so that an upper one 123a of said recesses engages with a lower one 133b of said protrusions so as to form a snap-fit lock removably sealing the clinical waste container 100 to the lid cap part 130 in an upper position. From this upper position, the lid cap part 130 may be pressed down further onto the lid part 120, but as long as the lid cap part 130 is not fully pressed down it is removable from the lid part 120 by simply pulling the lid cap part 130 upwards.

However, as mentioned the lid cap part 130 may be further arranged be pressed, from said upper position, further down onto to the lid part 120 to a lower position, in which an upper one 123a of said recesses engages with an upper one 133a of said protrusions and in which a lower one 123b of said recesses engages with a lower one 133b of said protrusions so as to form a snap-fit lock permanently sealing the clinical waste container 100 to the lid cap part 130.

In this lower position, it is preferred that a flap 135 of the lid cap part 130 is bent about 90° upwards, from a generally horizontal orientation to a generally vertical orientation, so as to signal that the clinical waste container 100 has been permanently sealed and should be reopened by brute force.

The lid part 120 comprise a angularly V elongated ditch or recess 126a running around the lower end 124 of the cylindric part 121. In the lower position, it is preferred that a lower end edge of the cylindric part 131 of the lip cap part 130 extends down into, and is received in, said ditch or recess 126a, at a vertical depth in said ditch or recess 126a so that said lower end edge of the cylindric part 131 is located beneath an upper surface of the cover 126 of the lid part 120. The vertical position of the lid cap part 130 in relation to the lid part 120 may be determined based on a relative orientation of the snap-lock means 123, 133 in relation to each other. This way, once the lid cap part 130 has been fully pushed down into the lower position, it is difficult to force the container 100 open again by inserting some kind of lever under the lid cap part 130 cylindric part 131 and bending it open.

At least one of said upper 133a and lower 133b protrusions may then be in the form of several separated elongated protrusions as discussed above, running in the angular direction V in relation to the lid cap part 130 and together defining a broken circle.

Said lower one 133b of said protrusions may be arranged to protrude radially R inwards between 0.3 and 0.5 mm, such as about 0.4 mm from an inner surface of the lid cap part 130.

Said upper one 133a of said protrusions may be arranged to protrude radially R inwards between 0.2 and 0.4 mm, such as about 0.3 mm. At any rate, it is preferred that the upper one 133a of the protrusions protrudes radially inwards R at least 0.05 mm less, such as at least 0.1 mm less, from said inner surface of the lid cap part 130 as compared to said lower one 133a of said protrusions.

As mentioned above, most or even substantially all of the lid part 120 may have a uniform material thickness. Such general material thickness of the lid part 120 may be at the most 2 mm, such as at the most 1.8 mm, such as between 1.5 and 1.7 mm, such as about 1.6 mm. The lid cap part 130 may have a similar or identical general material thickness as the lid part 120.

To the contrary, a corresponding general material thickness of the basket part 110 may be smaller than said general thickness of the lid part 120, such as at least 0.1 or even 0.2 mm smaller. The material thickness of the basket part 110 may be less than 1.7 mm, and it may be more than 1.5 mm. An exception could be in the bottom 113 of the basket part 110, where the material thickness can be larger, such as about 1.8 mm.

The present inventors have realised that using such general material thicknesses in the lid part 120 as compared to in the basket part 110 allows the optimisation of the use of wooden fibres globally in the clinical waste container 100 while still fulfilling the requirements given above. Specifically, the basket part 110 may be made from said plastic material comprising wooden fibres, but comprising a larger share of wooden fibres than the lid part 120 does. This means that the basket part 110 comprises a relatively smaller amount of plastic material (such as polypropylene). Since the basket part 110 contains a smaller volume of material, an overall optimised use of non-fibrous material is achieved, minimising the carbon footprint of the entire container 100.

Particularly in such cases, it is preferred that the basket part 110 comprises a horizontally protruding edge 112 running along its open upper end 111 and arranged to engage with corresponding snap-lock means 129 of the lid part 120 so as to achieve a snap-fit lock of the lid part 120 to the basket part 110, so as to in turn form the clinical waste container 100 in said operating orientation. Then, said horizontally protruding edge 112 has a minimum height of at least 1.5 mm, or even at least 2.0 mm.

As can be viewed in the Figures, the lid part 120 and the lid cap part 130 may be interconnected by a connecting part 140, constituting the same material body as the lid part 120 and the lid cap part 130. Hence, the lid part 120, the connecting part 140 and the lid cap part 130 are injected moulded into one and the same, integrated material body, without any joints. The connecting part 140 preferably has an elongated shape, that may stretch across at least 10 cm and with a maximum cross-sectional diameter of about 5 mm.

In preferred embodiments, the connecting part 140 is connected to a lower end of the lid part 120 and to a lower end of the lid cap part 130, when the clinical waste container 100 is in said upright operating orientation and the lid cap part 130 is mounted on the lid part 120, covering the lid opening 122, in which upright operating orientation the connecting part 140 is forced to bend (be bent) elastically at least 150° or, in the examples shown in the Figures, 180°, as a result of the lid cap part 130 being brought onto the lid opening 122.

As illustrated in the Figures, the connecting part 140 may be arranged to protrude horizontally from the lid part 120, at a proximal end 141 of the connecting part 140, and/or horizontally from the lid cap part 130, at a distal end 142 of the connecting part 140, when in a relaxed state and the clinical waste container 100 is in said upright operating orientation.

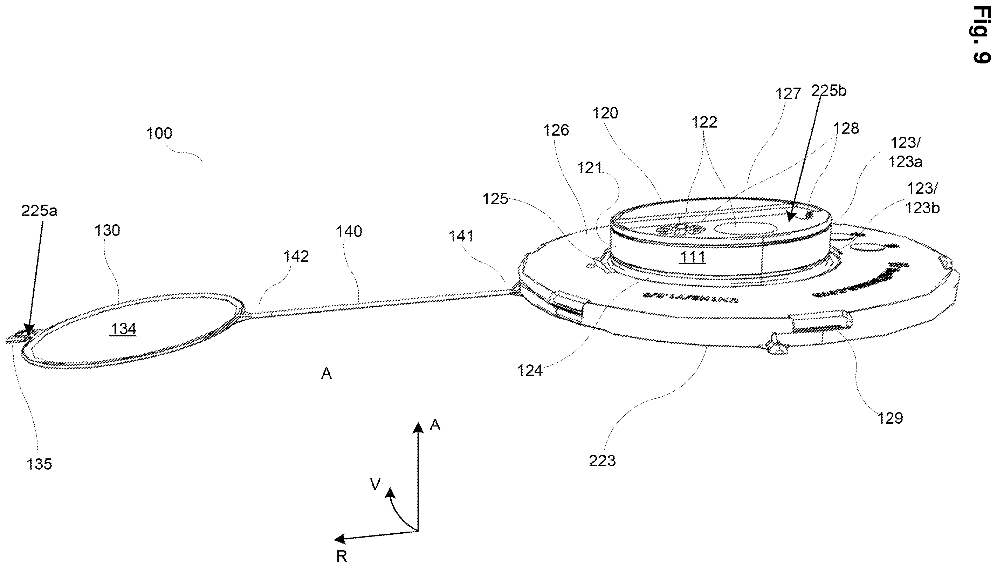

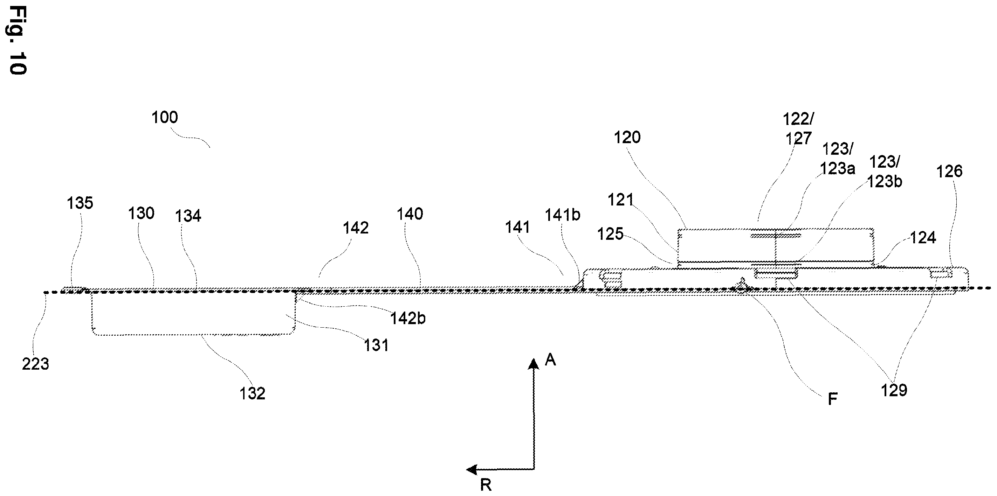

As illustrated in Figure 10, a contact plane 223 between a pair of mould parts 221, 222 used together to make the lid part 120 and the lid cap part 130 in an injection moulding process of the type described below, runs through said lower end of the lid cap part 130, through said lower end of the lid part 120 and through the connecting part 140, effectively splitting the material body in question in exactly two parts. The top one of these parts is shown in Figure 9. Using two injection locations, as described below, will efficiently fill such a structure with the presently used plastic material, in particular when using the material sizes and so forth described herein. The contact plane 223 may be planar, in other words not bent or curved.

As mentioned, the connecting part 140 may be elongated, and may in cross-section (the cross-section being perpendicular to a man longitudinal direction of the connecting part 140) comprise a pair of likewise elongated wing-like protrusions 143 extending on and out from either horizontal lateral sides of the connecting part 140 when the clinical waste container 100 is in said upright operating orientation. This is perhaps best seen in Figure 12.

In some embodiments, each of a connection point between the connecting part 140 and the lid part 120 (at said proximal end 141) and the lid cap part 130 (at said distal end 142), respectively, comprises two respective horizontal support structures 141a, 142a as well as a respective vertical support structure 141b, 142b.

Said horizontal support structures 141a, 142 each may extend in either lateral (horizontal in said operating orientation) direction in relation to the connecting part 140. Each of said support structures 141a, 141b, 142a, 142b may interconnect a respective lateral side of the connecting part 140 to an outside surface of the lid part 120 or lid cap part 130, respectively. Each of said support structures 141a, 141b, 142a, 142b may be triangular, as illustrated in the Figures, or have any other suitable shape. Preferably however, they interconnects a point at least 3 mm along the connecting part 140 end, from said side surface in question, with a point at least 3 mm from the connecting part 140 on said side surface in question, so as to achieve a strengthening of the connection between the connecting part 140 to the surface side (of the lid part 120 or the lid cap part 130) in question.

In particular in case the contact plane 223 is arranged in relation to the lid part 120 and the lid cap part 130 as has been described above, it is preferred that the vertical support part 141b interconnecting the connecting part 140 to the lid part 120 extends upwards from the connecting part 140, while the vertical support part 142b interconnecting the connecting part 140 to the lid cap part 130 extends downwards from the connecting part 140, when the clinic waste container 100 is in its operating orientation and in a relaxed state of the connecting part 140. See Figure 10.

This achieves a number of effects. Firstly, the structure can be efficiently filled during injection moulding. Secondly, the positioning of the contact plane 223 is optimal in terms of structural strength of both the lid part 120 and the lid cap part 130. Thirdly, the connection between the connecting part 140 and the lid part 120 and the lid cap part 130, respectively, is achieved that has sufficient strength to withstand the strain accruing when the connecting part 140 is bent to bringing the lid cap part 130 onto the cap part 120, even as it is manufactured from the above discussed relatively brittle material.

As shown in the Figures, the lid part 120 opening 122 may comprise an interface 127 for item disposal, that may for instance comprise a syringe needle or blade disconnector means 128, which in turn may be of standard type.

The Figures also show a fastening means F, in the form of a small head-shaped form. If a handle is needed to carry the container 100, a flexible string may be attached on either side of the lid part 120, to a respective such fastening means F.

The basket part 110 may hold a maximum volume of, for instance, at least 2 liters, and it may hold a maximum volume of, for instance, at the most 5 liters. By varying the height of the basket part 110, the total volume of the container 100 may be varied while using one and the same lid part 120 and one and the same lid cap part 130.

Figure 11 illustrates an alternative lid part 320 / lid cap part 330 / connecting part 340 configuration, that is generally similar to the configuration illustrated in Figures 1-10.

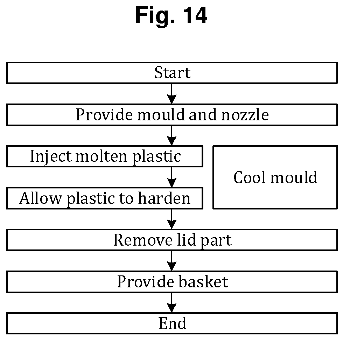

Figure 14 illustrates a method according to the present invention, for producing a clinical waste container 100 of the type described herein.

In a first step, the method starts.

In a subsequent step, a metal mould 220 is provided, together with a heated injection nozzle 210. The nozzle 210 may for part of the mould 220, and may be separately heated, such as using electric resistance cables arranged in or on the nozzle 210, as is conventional as such. The nozzle is arranged to delivery molten plastic material to a respective injection point in the mould. In case there are more than one such injection points, there is one nozzle per such injection point.

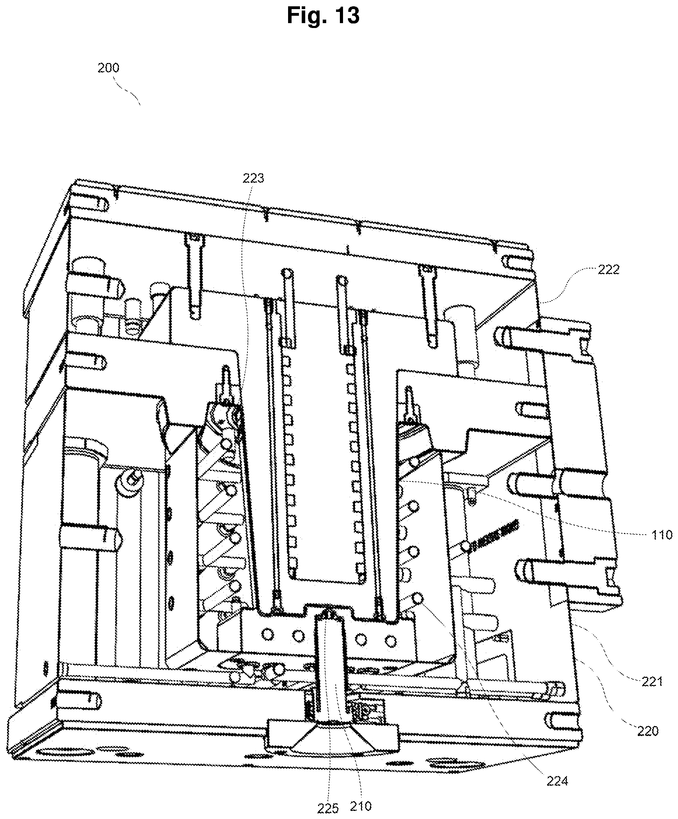

Figure 13 illustrates such a mould 220, with such an injection nozzle 210. It is realised that the exemplifying mould 220 shown in Figure 13 is a mould for producing the basket part 110, but a similar mould 220 will be used to produce either of the lid part 220, 420 variants.

For the lid part 220, 420, two injection points may be used, as will be described below.

In a subsequent step, molten plastic material is injected via the heated nozzle 210 into the mould 220, the molten plastic material comprising at least 30% by volume wooden fibres as described above.

In a subsequent step, the plastic material is allowed to harden by cooling to form said basket part 110, said lid part 120, said lid cap part 130, and/or said connecting part 140, as the case may be.

In a subsequent step, the part in question is removed from the mould 220. For the lid cap part 130, a per-se conventional ejector mechanism is used to eject the lid cap part 130 from the mould, the disjoint ridges of the inner snap-lock means 133 will not be damaged since they allow elastic deformation of the moulded material without breaking.

At least the lid part 120 and the lid cap part 130 is manufactured in this manner.

In a subsequent step, the basket part 110 is provided, potentially using injection moulding in the corresponding manner of the basket part 110.

In a subsequent step, the lid part 120 may be mounted onto the basket part 110 so as to form the final clinical waste container 100.

In a subsequent step, the method ends.

The present inventors have realised that good results are achieved in case said mould 220 and said heated nozzle 210 are both made from a stainless steel material comprising at least 12% chromium, such as about 13% chromium, and which stainless steel material is hardened to at least 50 HRC (Rockwell scale). One example of a suitable material is S50C 1.2083 steel. Such material will withstand both abrasive wear and the relatively aggressive chemical environment presented by the present molten material.

In the above-described preferred case in which the mould 220 defines the shape of the lid part 120 and the lid cap part 130 as a connected body being connected by the connecting part 140, and wherein both the lid part 120 and the lid cap part 130 are formed by said injection of said molten plastic material into said mould 220, the molten plastic may advantageously be injected into the mould 220 at two different injection points 225a, 225b (see Figure 9), one on the lid part 120 and another one on the lid cap part 130 (which may be at the flap 134).

As for the basket part 110, a single injection point 225 may be arranged in the bottom 113 of the basket part 110.

As discussed above, the contact plane 223 between the first mould part 221 and the second mould part 222 (the first 221 and second 222 mould parts forming said mould 220 in a mounted state) may be flat (planar) and may pass through said lid part 120, through said connecting part 140 and through said lid cap part 130.

In this case, the contact plane 223 may be horizontal when the lid part 120 is in said upright operating orientation. Also, the lid part 120 may in this case protrude mainly on a first side (an upper side in Figure 10) of the contact plane 223, whereas the lid cap part 130 may protrude mainly on a second side (a lower side in Figure 10) of the contact plane 223.

As is illustrated in Figure 13, the mould may contain integrated cooling channels 234, through which a cooling fluid (such as water) may be circulated so as to cool the mould 220 to a desired temperature during moulding.

The present inventors have realised that a relatively high cooling temperature is suitable to achieve the present goals. Hence, the method may further comprise actively and continuously cooling the mould 220, using said cooling fluid circulating in said channels 234 through the mould 220, during the hardening of the plastic material, to a constant temperature of at least 30°C, such as at least 40°C or even at least 50°C, during the whole moulding process.

Such a moulding process of a container 100, 300 of the above-described type, with the features described herein, achieves that full plastic injection can be achieved in a quick and efficient production process, resulting in a sufficiently durable container 100, 300 that achieves a significant lowering of its carbon footprint as compared to conventional clinical waste containers.

Above, preferred embodiments have been described. However, it is apparent to the skilled person that many modifications can be made to the disclosed embodiments without departing from the basic idea of the invention.

It is generally realised that the inventive effort underlying the present invention is to make a series of modifications to a conventional clinical waste container so as to allow the present biocomposite to be used instead of, for instance, 100% polypropylene. Herein, a number of such specific modifications have been described, and various ways of combining such modifications. It is, however, realised that the modifications and features described herein can be combined also in other ways, as the case may be.

Hence, the invention is not limited to the described embodiments, but can be varied within the scope of the enclosed claims.

## Claims

1. Clinical waste container (100) being associated, in an upright operating orientation of the clinical waste container (100), with an upwards axial direction (A), an outwards radial direction (R) and an angular direction (V), the clinical waste container (100) comprising
a lower basket part (110), having an upper basket opening (111);
an upper lid part (120), arranged to be fastened to said basket part (110) so as to cover said basket opening (111), the lid part (120) comprising a cylindrical part (121) having an upper lid opening (122) arranged to receive clinical waste through said lid opening (122) into said basket part (110); and
a lid cap part (130), arranged to be fastened to said lid part (120) so as to cover said lid opening (122), the lid cap part (130) comprising a cylindrical part (131) having a closed upper end (132) and an inner dimension being larger than an outer dimension of said cylindrical part (121) of the lid part (120), the cylindrical part (131) of the lid cap part (130) being arranged to be slid onto the cylindrical part (121) of the lid part (120) so that the lid cap part (130) as a result covers the lid opening (122),
wherein the cylindrical part (131) of the lid cap part (130) comprises inner snap-lock means (133) arranged to engage with outer snap-lock means (123) arranged on the cylindrical part (121) of the lid part (120) so as to fasten the lid cap part (130) on the lid part (120) by the material of at least the lid cap part (130) deforming elastically into a snap-fit lock,
**characterised in that**
the inner snap-lock means (133) comprises several separated elongated protrusions (133a,133b), running in the angular direction (V) in relation to the lid cap part (130) and together defining a broken circle, **in that**
the outer snap-lock means (123) comprises an elongated recess (123a,123b), running in the angular direction (V) in relation to the lid part (120) and being arranged to, by at least the material of the lid cap part (130) elastically deforming, engage with said protrusions (133a,133b) by said protrusions (133a,133b) protruding into said recess (123a,123b) along a respective angular (V) extension of said protrusions (133a,133b) and said recess (123a,123b), respectively, so as to form a first snap-fit lock, in turn fastening the lid cap part (130) to the lid part (120), **in that**
the lid part (120) comprises at a lower end (124) of its cylindrical part (121) a bend (125) at which the cylindrical part (121) of the lid part (120) changes direction at least 60° as seen in a cross-section including a centre axially (A) extending axis of the cylindrical part (121) of the lid part (120), the bend (125) having an inner radius of curvature (R1) of at the most 5 mm and an outer radius of curvature (R2) of at the most 5 mm, **in that**
the material thickness of the lid part (120) is at least 25% thicker locally at said bend (125) as compared to a material thickness adjacent to the bend (125) in said cylindrical part (121) of the lid part (120), and **in that**
the lid part (120) and the lid cap part (130) are made from a plastic material comprising at least 30% by volume wooden fibres.

2. Clinical waste container (100) according to claim 1, wherein
the material thickness of the lid part (120) at said bend (125) is locally varied by the inner radius of curvature (R1) being sufficiently large in relation to the outer radius of curvature (R2).

3. Clinical waste container (100) according to claim 2, wherein
an outside of the lid part (120) at said bend (125) has a part-circular shape in said cross-section, and wherein
an inside of the lid part (120) at said bend (125) has a part-circular shape in said cross-section.

4. Clinical waste container (100) according to any one of the preceding claims, wherein
the outer snap-lock means (123) comprises a pair of axially (A) offset but parallel elongated recesses (123a,123b) on the cylindrical part (121) of the lid part (120), wherein
the inner snap-lock means (133) comprises a pair of axially (A) offset but parallel elongated protrusions (133a,133b) on the cylindrical part (131) of the lid cap part (130), wherein
the lid cap part (130) is arranged to be pressed down onto the lid part (120) so that an upper one (123a) of said recesses engages with a lower one (133b) of said protrusions so as to form a snap-fit lock removably sealing the clinical waste container (100) to the lid cap part (130) in an upper position, wherein
the lid cap part (130) is further arranged be pressed, from said upper position, further down onto to the lid part (120) to a lower position, in which an upper one (123a) of said recesses engages with an upper one (133a) of said protrusions and in which a lower one (123b) of said recesses engages with a lower one (133b) of said protrusions so as to form a snap-fit lock permanently sealing the clinical waste container (100) to the lid cap part (130), and wherein
at least one of said upper (133a) and lower (133b) protrusion is in the form of several separated elongated protrusions, running in the angular direction (V) in relation to the lid cap part (130) and together defining a broken circle.

5. Clinical waste container (100) according to claim 4, wherein
said upper one (133a) of said protrusions is arranged to protrude radially (R) inwards at least 0.05 mm less from said inner surface of the lid cap part (130) as compared to said lower one (133a) of said protrusions.

6. Clinical waste container (100) according to any one of the preceding claims, wherein
a general material thickness of the lid part (120) is at the most 2 mm.

7. Clinical waste container (100) according to claim 6, wherein
a general material thickness of the basket part (110) is smaller than said general thickness of the lid part (120).

8. Clinical waste container (100) according to any one of the preceding claims, wherein
the basket part (110) is made from said plastic material but comprising a larger share of wooden fibres than the lid part (120).

9. Clinical waste container (100) according to any one of the preceding claims, wherein
the lid part (120) and the lid cap part (130) are interconnected by a connecting part (140), constituting the same material body as the lid part (120) and the lid cap part (130).

10. Clinical waste container (100) according to claim 9, wherein
the connecting part (140) is connected to a lower end of the lid part (120) and to a lower end of the lid cap part (130), when the clinical waste container (100) is in said upright operating orientation and the lid cap part (130) is mounted on the lid part (120), covering the lid opening (122), in which upright operating orientation the connecting part (140) is forced to be bent elastically at least 150° as a result of the lid cap part (130) being brought onto the lid opening (122).

11. Clinical waste container (100) according to claim 10, wherein
the connecting part (140) protrudes horizontally from the lid part (120) when in a relaxed state and the clinical waste container (100) is in said upright operating orientation.

12. Clinical waste container (100) according to claim 11, wherein
each of a connection point between the connecting part (140) and the lid part (120) and the lid cap part (130), respectively, comprises two respective horizontal support structures (141a,142a) each extending in either lateral direction in relation to the connecting part (140), as well as a respective vertical support structure (141b,142b), each of said support structures (141a,141b,142a,142b) interconnecting a respective side of the connecting part (140) to an outside of the lid part (120) or lid cap part (130), respectively, when the clinical waste container (100) is in said upright operating orientation, and wherein
the vertical support part (141b) interconnecting the connecting part (140) to the lid part (120) extends upwards from the connecting part (140) while the vertical support part (142b) interconnecting the connecting part (140) to the lid cap part (130) extends downwards from the connecting part (140).

13. Method for producing a clinical waste container (100) according to any one of preceding claims, wherein the method comprises
providing a metal mould (220) and a heated nozzle (210);
injecting molten plastic material comprising at least 30% by volume wooden fibres via said heated nozzle into said mould (220);
allowing the plastic material to harden by cooling to form said lid part (120);
removing the lid part (120) from the mould (220); and
providing said basket part (110).

14. Method according to claim 13, wherein
said mould (220) and said heated nozzle (210) are made from a stainless steel material comprising at least 12% chromium and is hardened to at least 50 HRC (Rockwell scale).

15. Method according to claim 13 or 14, wherein
said mould (220) defines the shape of the lid part (120) and said lid cap part (130) as a connected body being connected by a connecting part (140), both the lid part (120) and the lid cap part (130) being formed by said injection of said molten plastic material into said mould (220), and wherein
the molten plastic is injected into the mould (220) at two different injection points (225a,225b), one on the lid part (120) and another one on the lid cap part (130).

## Patentansprüche

1. Klinikabfallbehälter (100), dem in einer aufrechten Betriebsausrichtung des Klinikabfallbehälters (100) eine nach oben gerichtete axiale Richtung (A), eine nach außen gerichtete radiale Richtung (R) und eine Winkelrichtung (V) zugeordnet sind, wobei der Klinikabfallbehälter (100) umfasst
ein unteres Korbteil (110) mit einer oberen Korböffnung (111);
ein oberes Deckelteil (120), das dazu eingerichtet ist, an dem Korbteil (110) befestigt zu werden, um die Korböffnung (111) abzudecken, wobei das Deckelteil (120) ein zylindrisches Teil (121) mit einer oberen Deckelöffnung (122) umfasst, die dazu eingerichtet ist, Klinikabfälle durch die Deckelöffnung (122) in das Korbteil (110) aufzunehmen; und
ein Deckelkappenteil (130), das dazu eingerichtet ist, an dem Deckelteil (120) befestigt zu werden, um die Deckelöffnung (122) abzudecken, wobei das Deckelkappenteil (130) ein zylindrisches Teil (131) mit einem geschlossenen oberen Ende (132) und einer Innenabmessung umfasst, die größer als eine Außenabmessung des zylindrischen Teils (121) des Deckelteils (120) ist, wobei das zylindrische Teil (131) des Deckelkappenteils (130) dazu eingerichtet ist, auf das zylindrische Teil (121) des Deckelteils (120) aufgeschoben zu werden, sodass das Deckelkappenteil (130) dadurch die Deckelöffnung (122) abdeckt,
wobei das zylindrische Teil (131) des Deckelkappenteils (130) innere Schnappverschlussmittel (133) umfasst, die so eingerichtet sind, dass sie mit äußeren Schnappverschlussmitteln (123) in Eingriff gelangen, die an dem zylindrischen Teil (121) des Deckelteils (120) angeordnet sind, um das Deckelkappenteil (130) an dem Deckelteil (120) zu befestigen, indem sich das Material zumindest des Deckelkappenteils (130) elastisch in eine Schnappverriegelung verformt,
**dadurch gekennzeichnet, dass**
die inneren Schnappverschlussmittel (133) mehrere voneinander getrennte längliche Vorsprünge (133a, 133b) umfassen, die in der Winkelrichtung (V) relativ zu dem Deckelkappenteil (130) verlaufen und gemeinsam einen unterbrochenen Kreis definieren,
dass die äußeren Schnappverschlussmittel (123) eine längliche Aussparung (123a, 123b) umfassen, die in der Winkelrichtung (V) relativ zu dem Deckelteil (120) verläuft und so eingerichtet ist, dass sie durch elastische Verformung zumindest des Materials des Deckelkappenteils (130) mit den Vorsprüngen (133a, 133b) in Eingriff gelangt, wobei die Vorsprünge (133a, 133b) entlang einer jeweiligen Erstreckung in der Winkelrichtung (V) der Vorsprünge (133a, 133b) und der Aussparung (123a, 123b) in die Aussparung (123a, 123b) hineinragen, um eine erste Schnappverriegelung zu bilden, wodurch wiederum das Deckelkappenteil (130) an dem Deckelteil (120) befestigt wird,
dass das Deckelteil (120) an einem unteren Ende (124) seines zylindrischen Teils (121) eine Biegung (125) aufweist, an der das zylindrische Teil (121) des Deckelteils (120), betrachtet in einem Querschnitt, der eine mittlere, sich axial (A) erstreckende Achse des zylindrischen Teils (121) des Deckelteils (120) enthält, seine Richtung um mindestens 60° ändert, wobei die Biegung (125) einen inneren Krümmungsradius (R1) von höchstens 5 mm und einen äußeren Krümmungsradius (R2) von höchstens 5 mm aufweist,
dass die Materialdicke des Deckelteils (120) an der genannten Biegung (125) lokal mindestens 25 % größer ist als eine Materialdicke angrenzend an die Biegung (125) in dem genannten zylindrischen Teil (121) des Deckelteils (120), und
dass das Deckelteil (120) und das Deckelkappenteil (130) aus einem Kunststoffmaterial hergestellt sind, das mindestens 30 Vol.-% Holzfasern umfasst.

2. Klinikabfallbehälter (100) nach Anspruch 1, wobei
die Materialdicke des Deckelteils (120) an der genannten Biegung (125) dadurch lokal variiert ist, dass der innere Krümmungsradius (R1) im Verhältnis zu dem äußeren Krümmungsradius (R2) ausreichend groß ist.

3. Klinikabfallbehälter (100) nach Anspruch 2, wobei
eine Außenseite des Deckelteils (120) an der genannten Biegung (125) in dem genannten Querschnitt eine teilkreisförmige Form aufweist, und wobei
eine Innenseite des Deckelteils (120) an der genannten Biegung (125) in dem genannten Querschnitt eine teilkreisförmige Form aufweist.

4. Klinikabfallbehälter (100) nach einem der vorstehenden Ansprüche, wobei
die äußeren Schnappverschlussmittel (123) ein Paar axial (A) versetzter, jedoch paralleler länglicher Aussparungen (123a, 123b) an dem zylindrischen Teil (121) des Deckelteils (120) umfassen, wobei
die inneren Schnappverschlussmittel (133) ein Paar axial (A) versetzter, jedoch paralleler länglicher Vorsprünge (133a, 133b) an dem zylindrischen Teil (131) das Deckelkappenteil (130) umfassen, wobei
das Deckelkappenteil (130) dazu eingerichtet ist, nach unten auf das Deckelteil (120) gedrückt werden kann, sodass eine obere (123a) der genannten Aussparungen mit einem unteren (133b) der genannten Vorsprünge in Eingriff gelangt, um eine Schnappverriegelung zu bilden, die den Klinikabfallbehälter (100) lösbar mit dem sich in einer oberen Position befindenden Deckelkappenteil (130) verschließt, wobei
das Deckelkappenteil (130) ferner dazu eingerichtet ist, aus der genannten oberen Position weiter nach unten auf das Deckelteil (120) in eine untere Position gedrückt werden kann, in der eine obere (123a) der genannten Aussparungen mit einem oberen (133a) der genannten Vorsprünge in Eingriff gelangt und in der eine untere (123b) der genannten Aussparungen mit einem unteren (133b) der genannten Vorsprünge in Eingriff gelangt, um eine Schnappverriegelung zu bilden, die den Klinikabfallbehälter (100) dauerhaft mit dem Deckelkappenteil (130) verschließt, und wobei
mindestens einer von dem oberen (133a) und dem unteren (133b) Vorsprung in Form mehrerer voneinander getrennter länglicher Vorsprünge ausgebildet ist, die in der Winkelrichtung (V) relativ zu dem Deckelkappenteil (130) verlaufen und gemeinsam einen unterbrochenen Kreis definieren.

5. Klinikabfallbehälter (100) nach Anspruch 4, wobei
der obere (133a) der genannten Vorsprünge dazu eingerichtet ist, von der genannten Innenfläche des Deckelkappenteils (130) radial (R) nach innen um mindestens 0,05 mm weniger vorzustehen als der untere (133b) der genannten Vorsprünge.

6. Klinikabfallbehälter (100) nach einem der vorstehenden Ansprüche, wobei
eine allgemeine Materialdicke des Deckelteils (120) höchstens 2 mm beträgt.

7. Klinikabfallbehälter (100) nach Anspruch 6, wobei
eine allgemeine Materialdicke des Korbteils (110) geringer ist als die genannte allgemeine Materialdicke des Deckelteils (120).

8. Klinikabfallbehälter (100) nach einem der vorstehenden Ansprüche, wobei
das Korbteil (110) aus dem genannten Kunststoffmaterial hergestellt ist, jedoch einen größeren Anteil an Holzfasern als das Deckelteil (120) aufweist.

9. Klinikabfallbehälter (100) nach einem der vorstehenden Ansprüche, wobei
das Deckelteil (120) und das Deckelkappenteil (130) durch ein Verbindungsteil (140) miteinander verbunden sind, wobei das Verbindungsteil (140), das Deckelteil (120) und das Deckelkappenteil (130) denselben Materialkörper bilden.

10. Klinikabfallbehälter (100) nach Anspruch 9, wobei
das Verbindungsteil (140) mit einem unteren Ende des Deckelteils (120) und mit einem unteren Ende des Deckelkappenteils (130) verbunden ist, wenn sich der Klinikabfallbehälter (100) in der genannten aufrechten Betriebsausrichtung befindet und das Deckelkappenteil (130) auf dem Deckelteil (120) montiert ist und die Deckelöffnung (122) abdeckt, wobei das Verbindungsteil (140) in dieser aufrechten Betriebsausrichtung infolge des Aufbringens des Deckelkappenteils (130) auf die Deckelöffnung (122) gezwungen wird, sich um mindestens 150° elastisch zu biegen.

11. Klinikabfallbehälter (100) nach Anspruch 10, wobei
das Verbindungsteil (140) horizontal von dem Deckelteil (120) vorsteht, wenn es sich in einem entspannten Zustand befindet und sich der Klinikabfallbehälter (100) in der genannten aufrechten Betriebsausrichtung befindet.

12. Klinikabfallbehälter (100) nach Anspruch 11, wobei
jeder von einem Verbindungspunkt zwischen dem Verbindungsteil (140) und dem Deckelteil (120) und dem Deckelkappenteil (130) jeweils zwei horizontale Stützstrukturen (141a, 142a), die sich jeweils in eine der beiden seitlichen Richtungen in Bezug auf das Verbindungsteil (140) erstrecken, sowie jeweils eine vertikale Stützstruktur (141b, 142b) umfassen, wobei jede der genannten Stützstrukturen (141a, 141b, 142a, 142b) eine jeweilige Seite des Verbindungsteils (140) mit jeweils einer Außenseite des Deckelteils (120) und des Deckelkappenteils (130) verbindet, wenn sich der Klinikabfallbehälter (100) in der genannten aufrechten Betriebsausrichtung befindet, und wobei
die vertikale Stützstruktur (141b), die das Verbindungsteil (140) mit dem Deckelteil (120) verbindet, sich von dem Verbindungsteil (140) nach oben erstreckt, während sich die vertikale Stützstruktur (142b), die das Verbindungsteil (140) mit dem Deckelkappenteil (130) verbindet, von dem Verbindungsteil (140) nach unten erstreckt.

13. Verfahren zum Herstellen eines Klinikabfallbehälters (100) nach einem der vorstehenden Ansprüche, wobei das Verfahren umfasst:
Bereitstellen einer Metallgießform (220) und einer beheizten Düse (210);
Einspritzen geschmolzenen Kunststoffmaterials, das mindestens 30 Vol.-% Holzfasern umfasst, über die genannte beheizte Düse in die genannte Gießform (220);
Aushärtenlassen des Kunststoffmaterials durch Abkühlen, um das genannte Deckelteil (120) zu bilden;
Entnehmen des Deckelteils (120) aus der Gießform (220); und
Bereitstellen des genannten Korbteils (110).

14. Verfahren nach Anspruch 13, wobei
die genannte Gießform (220) und die genannte beheizte Düse (210) aus einem Edelstahlmaterial hergestellt sind, das mindestens 12 % Chrom umfasst und auf mindestens 50 HRC (Rockwell-Skala) gehärtet ist.

15. Verfahren nach Anspruch 13 oder 14, wobei
die genannte Gießform (220) die Form des Deckelteils (120) und des genannten Deckelkappenteil (130) als einen über ein Verbindungsteil (140) verbundenen Körper definiert, wobei sowohl das Deckelteil (120) und das Deckelkappenteil (130) durch das genannte Einspritzen des genannten geschmolzenen Kunststoffmaterials in die genannte Gießform (220) geformt werden, und wobei
der geschmolzene Kunststoff an zwei verschiedenen Einspritzpunkten (225a, 225b) in die Gießform (220) eingespritzt wird, von denen sich eine an dem Deckelteil (120) und die andere an dem Deckelkappenteil (130) befindet.

## Revendications

1. Récipient de déchets cliniques (100) qui est associé, dans une orientation de fonctionnement verticale du récipient de déchets cliniques (100), à une direction axiale vers le haut (A), à une direction radiale vers l'extérieur (R) et à une direction angulaire (V), le récipient de déchets cliniques (100) comprenant :
une partie de panier inférieure (110) ayant une ouverture de panier supérieure (111) ;
une partie de couvercle supérieure (120) agencée pour être fixée sur ladite partie de panier (110) afin de couvrir ladite ouverture de panier (111), la partie de couvercle (120) comprenant une partie cylindrique (121) ayant une ouverture de couvercle supérieure (122) agencée pour recevoir les déchets cliniques par ladite ouverture de couvercle (122) dans ladite partie de panier (110) ; et
une partie de capuchon de couvercle (130) agencée pour être fixée sur ladite partie de couvercle (120) afin de couvrir ladite ouverture de couvercle (122), la partie de capuchon de couvercle (130) comprenant une partie cylindrique (131) ayant une extrémité supérieure fermée (132) et une dimension interne qui est supérieure à une dimension externe de ladite partie cylindrique (121) de la partie de couvercle (120), la partie cylindrique (131) de la partie de capuchon de couvercle (130) étant agencée pour coulisser sur la partie cylindrique (121) de la partie de couvercle (120) de sorte que la partie de capuchon de couvercle (130) couvre par conséquent l'ouverture de couvercle (122),
dans lequel la partie cylindrique (131) de la partie de capuchon de couvercle (130) comprend un moyen d'encliquetage interne (133) agencé pour se mettre en prise avec le moyen d'encliquetage externe (123) agencé sur la partie cylindrique (121) de la partie de couvercle (120) afin de fixer la partie de capuchon de couvercle (130) sur la partie de couvercle (120) grâce au matériau d'au moins la partie de capuchon de couvercle (130) qui se déforme élastiquement dans un encliquetage,
**caractérisé en ce que** :
le moyen d'encliquetage interne (133) comprend plusieurs saillies allongées séparées (133a, 133b) s'étendant dans la direction angulaire (V) par rapport à la partie de capuchon de couvercle (130) et définissant ensemble un cercle brisé, **en ce que** :
le moyen d'encliquetage externe (123) comprend un évidement allongé (123a, 123b), s'étendant dans la direction angulaire (V) par rapport à la partie de couvercle (120) et étant agencé pour, grâce au moins au matériau de la partie de capuchon de couvercle (130) qui se déforme élastiquement, se mettre en prise avec lesdites saillies (133a, 133b) grâce auxdites saillies (133a, 133b) qui font saillie dans ledit évidement (123a, 123b) le long d'une extension angulaire (V) respective desdites saillies (133a, 133b) et dudit évidement (123a, 123b) respectivement, afin de former un premier encliquetage, fixant à son tour la partie de capuchon de couvercle (130) à la partie de couvercle (120), **en ce que** :
la partie de couvercle (120) comprend, au niveau d'une extrémité inférieure (124) de sa partie cylindrique (121), une courbure (125) au niveau de laquelle la partie cylindrique (121) de la partie de couvercle (120) change de direction d'au moins 60°, comme observé sur une section transversale comprenant un axe central s'étendant axialement (A) de la partie cylindrique (121) de la partie de couvercle (120), la courbure (125) ayant un rayon de courbure interne (R1) au maximum de 5 mm et un rayon de courbure externe (R2) au maximum de 5 mm, **en ce que** :
l'épaisseur de matériau de la partie de couvercle (120) est au moins 25% plus épaisse localement au niveau de ladite courbure (125) par rapport à une épaisseur de matériau adjacente à la courbure (125) dans ladite partie cylindrique (121) de la partie de couvercle (120), et **en ce que** :
la partie de couvercle (120) et la partie de capuchon de couvercle (130) sont constituées d'une matière plastique comprenant au moins 30% en volume de fibres de bois.

2. Récipient de déchets cliniques (100) selon la revendication 1, dans lequel :
l'épaisseur de matériau de la partie de couvercle (120) au niveau de ladite courbure (125) est localement modifiée par le rayon de courbure interne (R1) qui est suffisamment grand par rapport au rayon de courbure externe (R2).

3. Récipient de déchets cliniques (100) selon la revendication 2, dans lequel :
un extérieur de la partie de couvercle (120) au niveau de ladite courbure (125) a une forme partiellement circulaire dans ladite section transversale, et dans lequel :
un intérieur de la partie de couvercle (120) au niveau de ladite courbure (125) a une forme partiellement circulaire dans ladite section transversale.

4. Récipient de déchets cliniques (100) selon l'une quelconque des revendications précédentes, dans lequel :
le moyen d'encliquetage externe (123) comprend une paire d'évidements allongés axialement (A) décalés mais parallèles (123a, 123b) sur la partie cylindrique (121) de la partie de couvercle (120), dans lequel :
le moyen d'encliquetage interne (133) comprend une paire de saillies allongées axialement (A) décalées mais parallèles (133a, 133b) sur la partie cylindrique (131) de la partie de capuchon de couvercle (130), dans lequel :
la partie de capuchon de couvercle (130) est agencée pour être comprimée vers le bas sur la partie de couvercle (120) de sorte qu'un évidement supérieur (123a) desdits évidements se met en prise avec une saillie inférieure (133b) desdites saillies afin de former un encliquetage scellant, de manière amovible, le récipient de déchets cliniques (100) à la partie de capuchon de couvercle (130) dans une position supérieure, dans lequel :
la partie de capuchon de couvercle (130) est en outre agencée pour être comprimée davantage vers le bas, à partir de ladite position supérieure, sur la partie de couvercle (120), jusqu'à une position inférieure, dans laquelle un évidement supérieur (123a) desdits évidements se met en prise avec une saillie supérieure (133a) desdites saillies et dans laquelle un évidement inférieur (123b) desdits évidements se met en prise avec une saillie inférieure (133b) desdites saillies afin de former un encliquetage scellant, de manière permanente, le récipient de déchets cliniques (100) à la partie de capuchon de couvercle (130), et dans lequel :
au moins l'une parmi lesdites saillies supérieure (133a) et inférieure (133b) se présente sous la forme de plusieurs saillies allongées séparées, s'étendant dans la direction angulaire (V) par rapport à la partie de capuchon de couvercle (130) et définissant ensemble un cercle brisé.

5. Récipient de déchets cliniques (100) selon la revendication 4, dans lequel :
ladite saillie supérieure (133a) desdites saillies est agencée pour faire saillie radialement (R) vers l'intérieur d'au moins 0,05 mm de moins à partir de ladite surface interne de la partie de capuchon de couvercle (130) par rapport à ladite saillie inférieure (133a) desdites saillies.

6. Récipient de déchets cliniques (100) selon l'une quelconque des revendications précédentes, dans lequel :
une épaisseur générale de matériau de la partie de couvercle (120) est au maximum de 2 mm.

7. Récipient de déchets cliniques (100) selon la revendication 6, dans lequel :
une épaisseur générale de matériau de la partie de panier (110) est inférieure à ladite épaisseur générale de la partie de couvercle (120).

8. Récipient de déchets cliniques (100) selon l'une quelconque des revendications précédentes, dans lequel :
la partie de panier (110) est réalisée à partir de ladite matière plastique, mais comprenant une plus grande proportion de fibres de bois que la partie de couvercle (120).

9. Récipient de déchets cliniques (100) selon l'une quelconque des revendications précédentes, dans lequel :
la partie de couvercle (120) et la partie de capuchon de couvercle (130) sont interconnectées par une partie de raccordement (140), constituant le même corps de matériau que la partie de couvercle (120) et la partie de capuchon de couvercle (130).

10. Récipient de déchets cliniques (100) selon la revendication 9, dans lequel :
la partie de raccordement (140) est raccordée à une extrémité inférieure de la partie de couvercle (120) et à une extrémité inférieure de la partie de capuchon de couvercle (130), lorsque le récipient de déchets cliniques (100) est dans ladite orientation de fonctionnement verticale et que la partie de capuchon de couvercle (130) est montée sur la partie de couvercle (120), couvrant l'ouverture de couvercle (122), dans laquelle orientation de fonctionnement verticale, la partie de raccordement (140) est pliée de force, par voie élastique, à au moins 150°, en raison de la partie de capuchon de couvercle (130) qui a été amenée sur l'ouverture de couvercle (122).

11. Récipient de déchets cliniques (100) selon la revendication 10, dans lequel :
la partie de raccordement (140) fait horizontalement saillie de la partie de couvercle (120) lorsqu'elle est dans un état détendu et que le récipient de déchets cliniques (100) est dans ladite orientation de fonctionnement verticale.

12. Récipient de déchets cliniques (100) selon la revendication 11, dans lequel :
chacun parmi un point de raccordement entre la partie de raccordement (140) et la partie de couvercle (120) et la partie de capuchon de couvercle (130) respectivement, comprend deux structures de support horizontales (141a, 142a) respectives, s'étendant chacune dans l'une ou l'autre des directions latérales par rapport à la partie de raccordement (140), ainsi qu'une structure de support verticale (141b, 142b) respective, chacune desdites structures de support (141a, 141b, 142a, 142b) interconnectant un côté respectif de la partie de raccordement (140) à un extérieur de la partie de couvercle (120) ou de la partie de capuchon de couvercle (130) respectivement, lorsque le récipient de déchets cliniques (100) est dans ladite orientation de fonctionnement verticale et dans lequel :
la partie de support verticale (141b) interconnectant la partie de raccordement (140) à la partie de couvercle (120) s'étend vers le haut à partir de la partie de raccordement (140) alors que la partie de support verticale (142b) interconnectant la partie de raccordement (140) à la partie de capuchon de couvercle (130) s'étend vers le bas à partir de la partie de raccordement (140).

13. Procédé pour produire un récipient de déchets cliniques (100) selon l'une quelconque des revendications précédentes, dans lequel le procédé comprend les étapes consistant à :
prévoir un moule en métal (220) et une buse chauffée (210) ;
injecter une matière plastique en fusion comprenant au moins 30% en volume de fibres de bois via ladite buse chauffée dans ledit moule (220) ;
permettre à la matière plastique de durcir par refroidissement afin de former ladite partie de couvercle (120) ;
retirer la partie de couvercle (120) du moule (220) ; et
prévoir ladite partie de panier (110).

14. Procédé selon la revendication 13, dans lequel :
ledit moule (220) et ladite buse chauffée (210) sont réalisés à partir d'un matériau en acier inoxydable comprenant au moins 12% de chrome et est durci à au moins 50 HRC (échelle de Rockwell).

15. Procédé selon la revendication 13 ou 14, dans lequel :
ledit moule (220) définit la forme de la partie de couvercle (120) et de ladite partie de capuchon de couvercle (130) en tant que corps raccordé qui est raccordé par une partie de raccordement (140), la partie de couvercle (120) et la partie de capuchon de couvercle (130) étant toutes deux formées par ladite injection de ladite matière plastique en fusion dans ledit moule (220), et dans lequel :
le plastique en fusion est injecté dans le moule (220) à deux points d'injection (225a, 225b) différents, un sur la partie de couvercle (120) et l'autre sur la partie de capuchon de couvercle (130).
